# EUROPEAN PATENT SPECIFICATION

(11) **EP 0 689 447 B1**
(45) Date of publication and mention of the grant of the patent: **07.04.1999**
(21) Application number: 94910177.8
(22) Date of filing: 16.02.1994
(51) Int. Cl.: A61K 35/76

(54) **CYTOPATHIC VIRUSES FOR THERAPY AND PROPHYLAXIS OF NEOPLASIA**
CYTOPATHISCHE VIREN ZUR THERAPIE UND PROPHYLAXE DER NEOPLASIE
VIRUS CYTOPATHIQUE POUR LE TRAITEMENT ET LA PROPHYLAXIE DE LA NEOPLASIE

(30) Priority: 16.02.1993 US 17525
(43) Date of publication of application: 03.01.1996
(60) Divisional application: 98121753.2
(73) Proprietor: ONYX PHARMACEUTICALS, Richmond, CA 94806-5206 (US)
(72) Inventor: McCORMICK, Francis, Richmond, CA 94804 (US)
(74) Representative: Greaves, Carol Pauline
(86) International application number: US9402049
(87) International publication number: WO9418992

(56) References cited:
- EP-A- 0 514 603
- Science, Vol. 252, issued 10 May 1991, MARTUZA et al., "Experimental Therapy of Human Glioma by Means of a Genetically Engineered Virus Mutant", pages 854-856, see entire document.
- Journal of General Virology, Vol. 72, issued 1991, SHINGU et al., "Therapeutic Effects of Bovine Enterovirus Infection on Rabbits with Experimentally Induced Adult T Cell Leukaemia", pages 2031-2034.
- FIELDS et al., "Virology", published 1985 by Raven Press (N.Y.), pages 477-495.
- Molecular and Cellular Biology, Vol. 8, No. 5, issued May 1988, VELCICH et al., "Adenovirus E1a ras Cooperation Activity is Separate from Its Positive and Negative Transcription Regulatory Functions", pages 2177-2183.
- Virology, Vol. 176, issued 1990, MAK et al., "Separate Regions of an Adenovirus E1B Protein Critical for Different Biological Functions", pages 553-562.

## Description

### TECHNICAL FIELD

The invention provides compositions of recombinant cytopathic viruses which are capable of replication and/or expression of late region genes in neoplastic mammalian cells but are essentially non-replicable in non-neoplastic cells, methods for constructing and propagating such recombinant viruses, methods for treating neoplastic disease with such recombinant viruses, and therapeutic compositions comprising such recombinant viruses.

### BACKGROUND

The proliferation of normal cells is thought to be regulated by growth-promoting proto-oncogenes counterbalanced by growth-constraining tumor-suppressor genes. Mutations that potentiate the activities of proto-oncogenes create the oncogenes that force the growth of neoplastic cells. Conversely, genetic lesions that inactivate tumor suppressor genes, generally through mutation(s) that lead to a cell being homozygous for the inactivated tumor suppressor allele, can liberate the cell from the normal replicative constraints imposed by these genes. Usually, an inactivated tumor suppressor gene (e.g., *p53*, *RB*, *DCC*, *NF-1*) in combination with the formation of an activated oncogene (i.e., a proto-oncogene containing an activating structural or regulatory mutation) can yield a neoplastic cell capable of essentially unconstrained growth (i.e., a transformed cell).

Oncogenic transformation of cells leads to a number of changes in cellular metabolism, physiology, and morphology. One characteristic alteration of oncogenically transformed cells is a loss of responsiveness to constraints on cell proliferation and differentiation normally imposed by the appropriate expression of cell- growth regulatory genes.

While different types of genetic alterations may all lead to altered expression or function of cell-growth regulatory genes and to abnormal growth, it is generally believed that more than one event is required to lead to neoplastic transformation of a normal cell to a malignant one (Land et al. (1983) Nature 304: 596; Weinberg RA (1989) Cancer Res. 49: 3713). The precise molecular pathways and secondary changes leading to malignant transformation for most cell types are not clear. A number of cases have been reported in which altered expression or activity of some proteins with putative cell-cycle control functions and/or implicated in the formation of functional transcriptional complexes, such as *p53* and *RB*, can lead to loss of proliferation control in cells (Ullrich et al. (1992) J. Biol. Chem. 267: 15259; Hollstein et al. (1991) Science 253: 49; Sager R (1992) Curr. Opin. Cell. Biol. 4: 155; Levine et al. (1991) Nature 351: 453).

Some oncogenes have been found to possess characteristic activating mutations in a significant fraction of certain cancers. For example, particular mutations in the *ras*^{*H*} and *ras*^{*K*} coding regions (e.g., codon 12, codon 61; Parada et al. (1984) Nature 312: 649) and the *APC* gene (Powell et al. (1992) Nature 359: 235) are associated with oncogenic transformation of cultured cells and are present in a striking percentage of specific human cancers (e.g., colon adenocarcinoma, bladder carcinoma, lung carcinoma and adenocarcinoma, hepatocarcinoma). These findings have led to the development of diagnostic and therapeutic reagents (e.g., polynucleotide probes and antibodies) that specifically recognize the activated form(s) of such oncogenes (U.S. Patent 4,798,787 and U.S. Patent 4,762,706).

The excessive or inappropriate expression of other oncogenes, such as *myc*, *erbB-2*, and *pim-1*, appears to be able to potentiate oncogenic transformation without necessarily requiring the presence of activating mutation(s) in the coding region. Overexpression of *erbB-2* is frequently found in adenocarcinoma of the breast, stomach, and ovary, and *erbB-2* levels in these cell types might serve as a diagnostic marker for neoplasia and/or may correlate with a specific tumor phenotype (e.g., resistance to specific drugs, growth rate, differentiation state).

Transgenic animals harboring various oncogenes (U.S. Patent 4,736,866 and U.S. Patent 5,087,571) or functionally disrupted tumor suppressor genes (Donehower et al. (1992) Nature 356: 215) have been described for use in carcinogen screening assays, among other potential uses.

Despite this progress in developing a more defined model of the molecular mechanisms underlying the transformed phenotype and neoplasia, few significant therapeutic methods applicable to treating cancer beyond conventional chemotherapy have resulted. Many conventional chemotherapeutic agents have a low therapeutic index, with therapeutic dosage levels being at or near dosage levels which produce toxicity. Toxic side effects of most conventional chemotherapeutic agents are unpleasant and lead to life-threatening bone marrow suppression, among other side effects.

Recent approaches for performing gene therapy to correct or supplement defective alleles which cause congenital diseases, such as cystic fibrosis, have been attempted with reports of limited initial success. Some gene therapy approaches involve transducing a polynucleotide sequence capable of expressing a functional copy of a defective allele into a cell in vivo using replication-deficient recombinant adenovirus (Rosenfeld et al. (1992) Cell 68: 143). Some of these gene therapy methods are efficient at transducing polynucleotides into isolated cells explanted from a patient, but have not been shown to be highly efficient in vivo. Therapeutic approaches to cancer which rely on transfection of explanted tumor cells with polynucleotides encoding tumor necrosis factor (TNF) and interleukin-2 (IL-2) have been described (Pardoll D (1992) Curr. Opin. Oncol. 4: 1124).

Although it might someday prove possible for gene therapy methods to be adapted to correct defective alleles of oncogenes or tumor suppressor genes in transformed cells in vivo, present gene therapy methods have not been reported to be able to efficiently transduce and correctly target (e.g., by homologous recombination) a sufficient percentage of neoplastic cells for practical gene therapy of neoplasia in situ. The nature of cancer biology mandates that a substantial fraction of the neoplastic cells, preferably all of the clonal progeny of the transformed cell, are ablated for an effective therapeutic effect. Moreover, present methods for gene therapy are very expensive, requiring ex vivo culturing of explanted cells prior to reintroduction into a patient. Widespread application of such methods, even if they were effective, would be prohibitively expensive.

Thus there exists a need in the art for methods and compositions for diagnosis and therapy of neoplastic diseases, especially for methods which selectively ablate neoplastic cells without the undesirable killing of non-neoplastic cells that is typical of conventional antineoplastic chemotherapy. The present invention fulfills these and other needs.

The references discussed herein are provided solely for their disclosure prior to the filing date of the present application.

### SUMMARY OF THE INVENTION

The present invention provides several novel methods and compositions for ablating neoplastic cells by infecting the neoplastic cells with a recombinant adenovirus which is substantially replication deficient in non-neoplastic cells and which exhibits at least a partial replication phenotype in neoplastic cells. The difference in replication phenotype of the adenovirus constructs of the invention in neoplastic and non-neoplastic cells provides a biological basis for viral-based therapy of cancer. Expression of adenoviral cytopathic effects, and optionally expression of a negative-selectable drug gene (e.g., HSV *tk*), are correlated with the adenoviral replication phenotype characteristic of neoplastic cells infected with the recombinant adenovirus constructs of the invention, thus discriminating between neoplastic and non-neoplastic cells and providing selective cytotoxicity of neoplastic cells. Although the methods are described in detail specifically for adenoviral constructs, the methods are believed to be applicable to essentially any virus type wherein efficient replication requires binding and/or sequestration and/or inactivation of a host cell protein that is present in non-neoplastic cells but is substantially absent or nonfunctional in neoplastic cells (e.g., *p53*, *RB*).

In order for adenovirus to replicate efficiently in cells, the adenoviral E1b gene product, p55, forms a complex with the host cell *p53* protein, thereby sequestering and/or inactivating *p53* and producing a cell that is deficient in *p53* function. Such a cell made deficient in *p53* function can support replication of the adenovirus. In this way, wild-type adenovirus is able to replicate in cells containing *p53*, as the adenovirus p55 proteins inactivates and/or sequesters the host cell *p53* protein.

The present invention thus provides a recombinant replication deficient adenovirus lacking an expressed viral oncoprotein capable of binding a functional p53 tumour suppressor gene product, for use in a method of treatment or therapy of a mammal; said treatment or therapy comprising the ablation of neoplastic cells in a cell population comprising said neoplastic cells and non-neoplastic cells; said non-neoplastic cells containing a functional p53 tumour suppressor gene product capable of forming a bound complex with said viral oncoprotein and said neoplastic cells lacking said functional p53 tumour suppressor gene product. The invention further provides a pharmaceutical composition comprising a recombinant replication deficient adenovirus of the invention and a pharmaceutically acceptable carrier.

The invention also provides use of a recombinant replication deficient adenovirus lacking an expressed viral oncoprotein capable of binding a functional p53 tumour suppressor gene product, for the manufacture of a medicament for the ablation of neoplastic cells in a cell population comprising said neoplastic cells and non-neoplastic cells; said non-neoplastic cells containing a functional p53 tumour suppressor gene product capable of forming a bound complex with said viral oncoprotein and said neoplastic cells lacking said functional p53 tumour suppressor gene product.

In another aspect, the invention provides an *in vitro* method of ablating neoplastic cells lacking a functional p53 tumour suppressor gene product in a cell population of neoplastic and non-neoplastic cells which have p53 tumour suppressor function, said method comprising: contacting under infective conditions (1) a recombinant replication deficient adenovirus lacking an expressed viral oncoprotein capable of binding a functional p53 tumour suppressor gene product, with (2) a cell population comprising said neoplastic cells and non-neoplastic cells, thereby generating an infected cell population such that ablation of said neoplastic cells occurs.

The invention also provides an *in vitro* method for detecting the absence of functional tumour suppressor activity in a cell population, comprising: contacting said cell population with a recombinant replication deficient adenovirus lacking an expressed viral oncoprotein capable of binding a functional p53 tumour suppressor gene product, under infective conditions, and determining the number or proportion of cells in said population with a replication phenotype.

In one embodiment of the invention, a recombinant adenovirus comprising an E1bₒlocus encoding a mutant p55 protein that is substantially incapable of forming a functional complex with *p53* protein in infected cells is administered to an individual or cell population comprising a neoplastic cell capable of being infected by the recombinant adenovirus. The substantial incapacity of the recombinant adenovirus to effectively sequester *p53* protein in infected non-neoplastic cells results in the introduced recombinant adenoviral polynucleotide(s) failing to express a replication phenotype in non-neoplastic cells. By contrast, neoplastic cells which lack a functional *p53* protein support expression of a replication phenotype by the introduced recombinant adenovirus which leads to ablation of the neoplastic cell by an adenoviral cytopathic effect and/or expression of a negative selection gene linked to the replication phenotype. In preferred variations of these embodiments, the recombinant adenovirus comprises an E1b locus encoding a mutant p55 which is substantially incapable of binding *p53* and may optionally also lack a functional p19 protein (i.e., incapable of inhibiting expression of adenoviral early region genes in the presence of E1a polypeptides). Recombinant adenoviruses of the invention may further comprise a mutant p19 gene which produces enhanced cytopathic effects; such a mutant known in the art is the p19 *cyt* mutant gene. However, it may be preferable to retain functional p19 in some mutants to maintain the integrity of viral DNA during the infection.

In a further embodiment of the invention, the recombinant adenovirus may also comprise an E1a locus encoding an E1a protein (e.g., p289R or p243R) that is substantially incapable of forming a complex with *RB* protein in infected cells is administered to an individual or cell population comprising a neoplastic cell capable of being infected by the recombinant adenovirus. The substantial incapacity of the recombinant adenovirus to effectively sequester *RB* protein in infected non-neoplastic cells results in the introduced recombinant adenoviral polynucleotide(s) failing to express a replication phenotype in non-neoplastic cells. By contrast, neoplastic cells which lack a functional *RB* protein support expression of a replication phenotype by the introduced recombinant adenovirus which leads to ablation of the neoplastic cell by an adenoviral cytopathic effect and/or expression of a negative selection gene linked to the replication phenotype. In preferred variations of these embodiments, the recombinant adenovirus comprises an E1a locus encoding a mutant E1a protein (e.g., p289R) that lacks a CR1 and/or CR2 domain capable of binding *RB* (and/or the 300kD polypeptide and/or the 107kD polypeptide) but comprises a functional CR3 domain capable of transactivation of adenoviral early genes. Additional variations of these embodiments include those where the recombinant adenovirus comprises a nonfunctional E1a locus which is substantially incapable of expressing a protein that binds to and inactivates *RB* and may optionally also comprise a functional p19 protein (i.e., capable of stimulating expression of adenoviral early region genes in the absence of E1a function). Recombinant adenoviruses of the invention may further comprise a mutant p19 gene which produces enhanced cytopathic effects; such a mutant known in the art is the p19 *cyt* mutant gene.

The invention provides novel recombinant adenovirus constructs which are replication defective in non-neoplastic cells but capable of expressing a replication phenotype in neoplastic cells lacking functional *p53* and optionally also lacking functional *RB*. The novel recombinant adenovirus constructs comprise a mutation, such as a deletion or point mutation, in the E1a and/or E1b gene regions, especially in the sequences encoding the E1b p55 protein and the CR1 and CR2 domains of the E1a p289R or p243R proteins. In some embodiments, a negative selectable gene, such as an HSV *tk* gene, is operably linked to an early region (e.g., E2, E1a, E1b) enhancer/promoter, a late region gene enhancer/promoter (e.g., major late promoter), or an early or late region promoter with a CMV enhancer, in a recombinant adenovirus construct also comprising an E1a or E1b mutation, so that the negative selectable gene is preferentially transcribed in infected cells which express a replication phenotype (i.e., neoplastic cells) and provides negative selection of such cells by administration of an effective dosage of a negative selection agent (e.g., gancyclovir, FIAU). A negative selectable gene may be inserted in place of an E1a and/or E1b structural sequence to concomitantly form an E1a⁽⁻⁾ replication deficient mutant, E1b⁽⁻⁾ replication deficient mutant, or E1a/E1b double mutant, respectively.

Antineoplastic compositions comprising such recombinant adenovirus in a pharmaceutically acceptable form for delivery to a neoplastic cell population in vivo are also provided.

The invention allows the provision of novel methods for treating a neoplastic disease comprising the steps of administering to a patient a recombinant virus capable of preferentially expressing a replication phenotype and/or expressing a cytopathic effect in a neoplastic cell population as compared to expression in a non-neoplastic cell population.

### BRIEF DESCRIPTION OF THE DRAWING

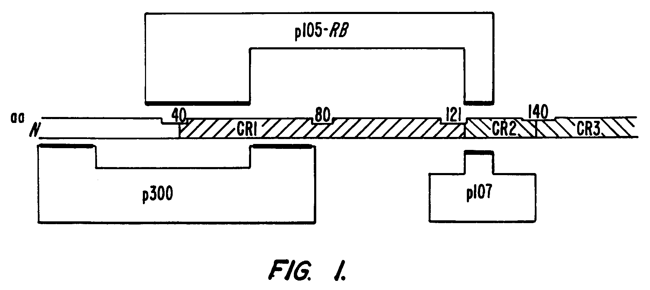

Fig. 1 shows a schematic representation of the domain structure and protein-protein interactions of an adenovirus E1a-289R polypeptide.

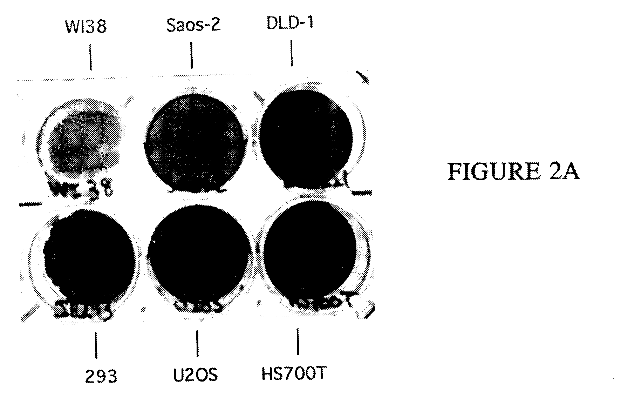

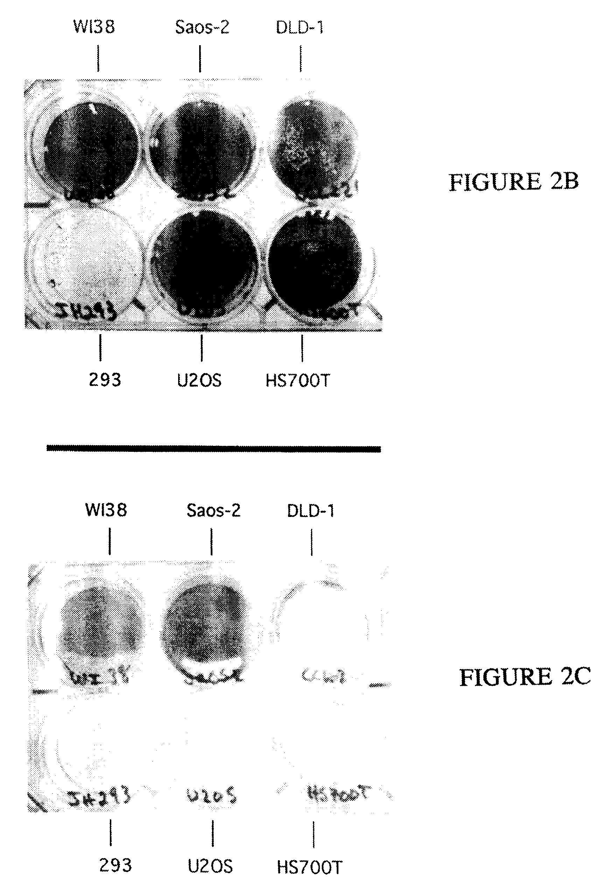

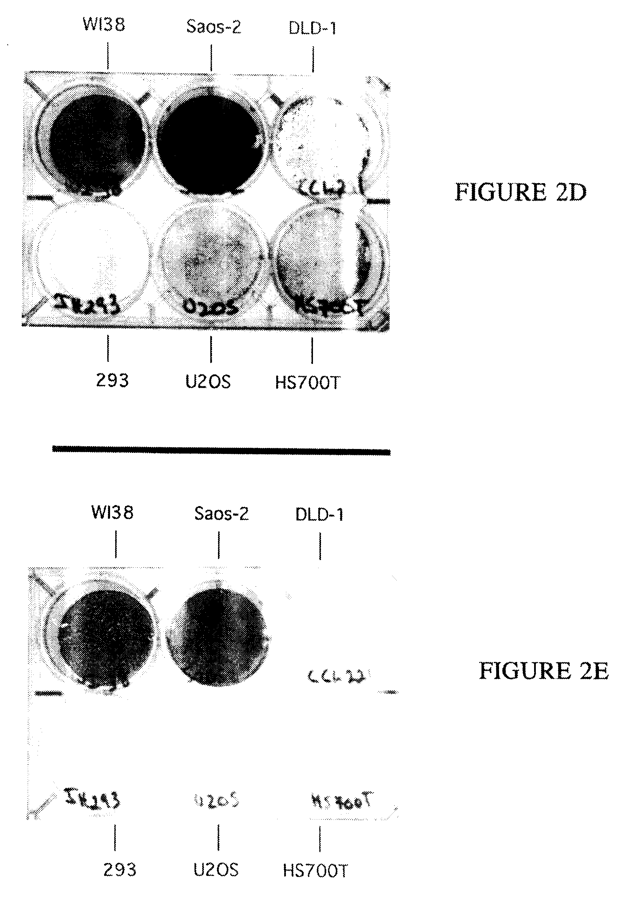

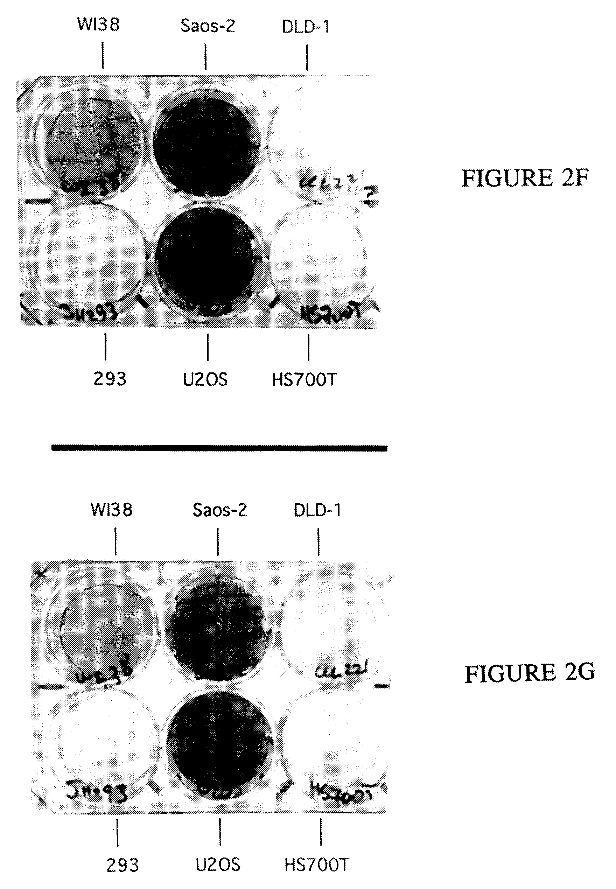

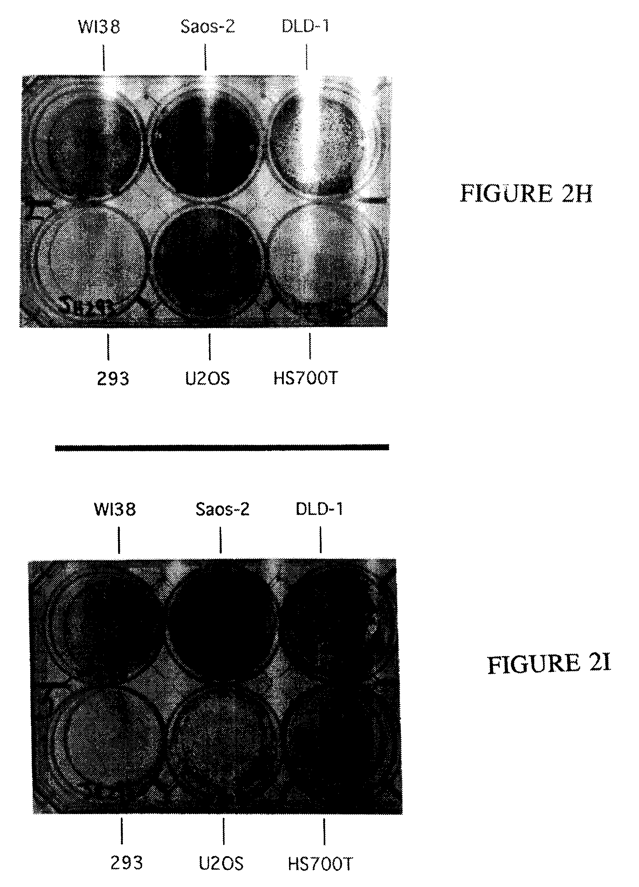

Fig. 2(a-i) shows infection of normal and neoplastically transformed human cell lines with wild-type adenovirus and recombination-deficient recombinant adenovirus mutants. Each cell line indicated was mock-infected, Fig. 2(a); or infected at an MOI of 1.0 with wild-type adenovirus 2, Fig. 2(b-c); mutant derivative dl 1010, Fig. 2(d-e); mutant derivative dl 1520, Fig. 2(f-g); or mutant derivative dl 434, Fig. 2(h-i). Fig. 2(a) shows results 9 days after the mock infection. The remaining panels show results from the following post-infection periods: Fig. 2(b), 4 days; Fig. 2(c), 7 days; Fig. 2(d), 8 days; Fig. 2(e), 12 days; Fig. 2(f), 14 days; Fig. 2(g), 20 days; Fig. 2(h), 6 days; Fig. 2(i), 14 days. On various days post-infection (noted *supra*), the cells were washed with PBS to remove dead cells and the remaining cells were stained with crystal violet. Each cell line mock infected with PBS alone was included as a control for viability.

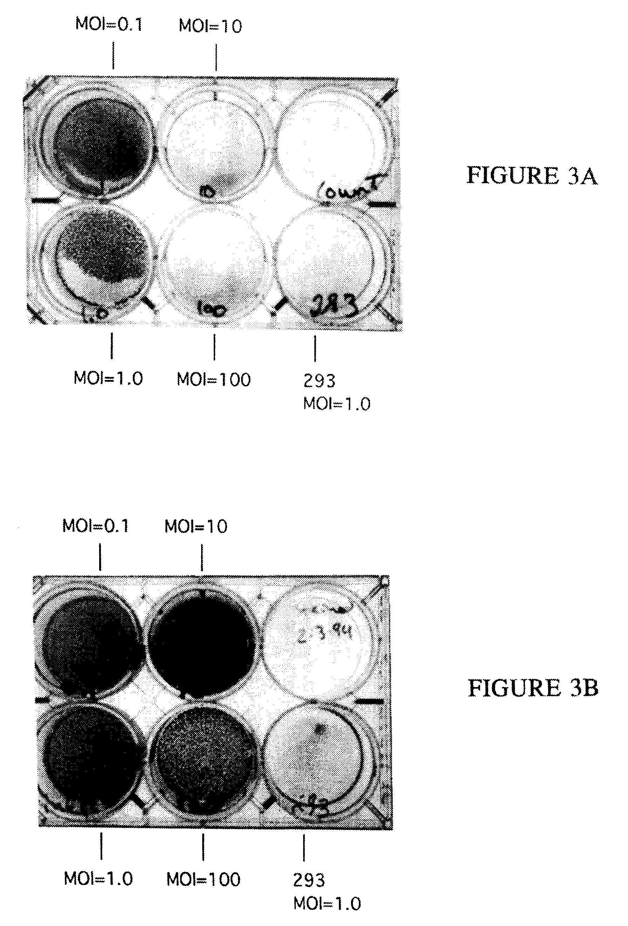

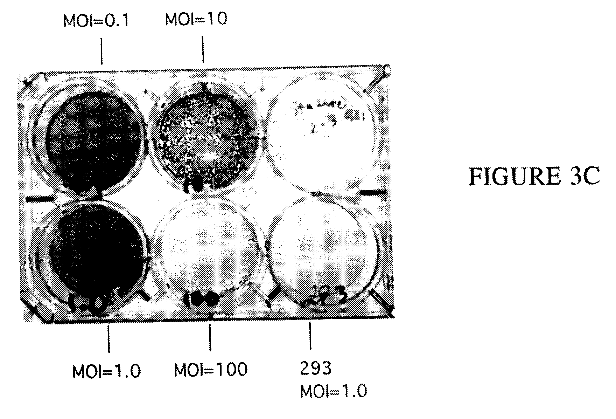

Fig. 3(a-c) shows infection of the normal human lung diploid fibroblast cell line IMR90 (ATCC CCL 186) with wild-type and mutant adenoviruses. IMR90 cells were infected with the indicated virus at MOIs corresponding to 0.1, 1.0, 10, and 100. At the indicated days post-infection, cells were washed with PBS and stained with crystal violet. One well in each 6-well dish was seeded with 293 cells to serve as a positive control for virus infection. Fig. 3(a) shows cells infected with wild-type Ad2 at 9 days post-infection. Fig. 3(b) shows cells infected with dl 1010 at 9 days post-infection. Fig. 3(c) shows cells infected with dl 1520 at 9 days post-infection.

### Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are described. For purposes of the present invention, the following terms are defined below.

The term "naturally-occurring" as used herein as applied to an object refers to the fact that an object can be found in nature. For example, a polypeptide or polynucleotide sequence that is present in an organism (including viruses) that can be isolated from a source in nature and which has not been intentionally modified by man in the laboratory is naturally-occurring. As used herein, the term "recombinant" indicates that a polynucleotide construct (e.g., and adenovirus genome) has been generated, in part, by intentional modification by man.

As used herein, the term "replication deficient virus" refers to a virus that preferentially inhibits cell proliferation or induces apoptosis in a predetermined cell population (e.g., cells substantially lacking *p53* and optionally also lacking *RB* function) which supports expression of a virus replication phenotype, and which is substantially enable to inhibit cell proliferation, induce apoptosis, or express a replication phenotype in cells comprising normal *p53* and optionally also *RB* function levels characteristic of non-replicating, non-transformed cells. Typically, a replication deficient virus exhibits a substantial decrease in plaquing efficiency on cells comprising normal *p53* and optionally also *RB* function.

As used herein, the term "*p53* function" refers to the property of having an essentially normal level of a polypeptide encoded by the *p53* gene (i.e., relative to non-neoplastic cells of the same histological type), wherein the *p53* polypeptide is capable of binding an E1b p55 protein of wild-type adenovirus 2 or 5. For example, *p53* function may be lost by production of an inactive (i.e., mutant) form of *p53* or by a substantial decrease or total loss of expression of *p53* polypeptide(s). Also, *p53* function may be substantially absent in neoplastic cells which comprise *p53* alleles encoding wild-type *p53* protein; for example, a genetic alteration outside of the *p53* locus, such as a mutation that results in aberrant subcellular processing or localization of *p53* (e.g., a mutation resulting in localization of *p53* predominantly in the cytoplasm rather than the nucleus) can result in a loss of *p53* function.

As used herein, the term "*RB* function" refers to the property of having an essentially normal level of a polypeptide encoded by the *RB* gene (i.e., relative to non-neoplastic cells of the same histological type), wherein the *RB* polypeptide is capable of binding an E1a protein of wild-type adenovirus 2 or 5. For example, *RB* function may be lost by production of an inactive (i.e., mutant) form of *RB* or by a substantial decrease or total loss of expression of *RB* polypeptide(s). Also, *RB* function may be substantially absent in neoplastic cells that comprise *RB* alleles encoding a wild-type *RB* protein; for example, a genetic alteration outside of the *RB* locus, such as a mutation that results in aberrant subcellular processing or localisation of *RB*, may result in a loss of *RB* function.

As used herein, the term "replication phenotype" refers to one or more of the following phenotypic characteristics of cells infected with a virus such as a replication deficient adenovirus: (1) substantial expression of late gene products, such as capsid proteins (e.g., adenoviral penton base polypeptide) or RNA transcripts initiated from viral late gene promoter(s), (2) replication of viral genomes or formation of replicative intermediates, (3) assembly of viral capsids or packaged virion particles, (4) appearance of cytopathic effect (CPE) in the infected cell, (5) completion of a viral lytic cycle, and (6) other phenotypic alterations which are typically contingent upon abrogation of *p53* or *RB* function in non-neoplastic cells infected with a wild-type replication competent DNA virus encoding functional oncoprotein(s). A replication phenotype comprises at least one of the listed phenotypic characteristics, preferably more than one of the phenotypic characteristics.

The term "antineoplastic replication deficient virus" is used herein to refer to a recombinant virus which has the functional property of inhibiting development or progression of a neoplasm in a human, by preferential cell killing of infected neoplastic cells relative to infected non-replicating, non-neoplastic cells of the same histological cell type.

As used herein, "neoplastic cells" and "neoplasia" refer to cells which exhibit relatively autonomous growth, so that they exhibit an aberrant growth phenotype characterized by a significant loss of control of cell proliferation. Neoplastic cells comprise cells which may be actively replicating or in a temporary non-replicative resting state (G₁ or G₀); similarly, neoplastic cells may comprise cells which have a well-differentiated phenotype, a poorly-differentiated phenotype, or a mixture of both type of cells. Thus, not all neoplastic cells are necessarily replicating cells at a given timepoint. The set defined as neoplastic cells consists of cells in benign neoplasms and cells in malignant (or frank) neoplasms. Frankly neoplastic cells are frequently referred to as cancer, typically termed carcinoma if originating from cells of endodermal or ectodermal histological origin, or sarcoma if originating from cell types derived from mesoderm.

As used herein, the term "operably linked" refers to a linkage of polynucleotide elements in a functional relationship. A nucleic acid is "operably linked" when it is placed into a functional relationship with another nucleic acid sequence. For instance, a promoter or enhancer is operably linked to a coding sequence if it affects the transcription of the coding sequence. Operably linked means that the DNA sequences being linked are typically contiguous and, where necessary to join two protein coding regions, contiguous and in reading frame. However, since enhancers generally function when separated from the promoter by several kilobases and intronic sequences may be of variable lengths, some polynucleotide elements may be operably linked but not contiguous.

As used herein, "physiological conditions" refers to an aqueous environment having an ionic strength, pH, and temperature substantially similar to conditions in an intact mammalian cell or in a tissue space or organ of a living mammal. Typically, physiological conditions comprise an aqueous solution having about 150 mM NaCl (or optionally KCl), pH 6.5-8.1, and a temperature of approximately 20-45° C. Generally, physiological conditions are suitable binding conditions for intermolecular association of biological macromolecules. For example, physiological conditions of 150 mM NaCl, pH 7.4, at 37°C are generally suitable.

### DETAILED DESCRIPTION

Generally, the nomenclature used hereafter and the laboratory procedures in cell culture, molecular genetics, and molecular virology described below are those well known and commonly employed in the art. Standard techniques are used for recombinant nucleic acid methods, polynucleotide synthesis, polypeptide synthesis, generation and propagation of virus stocks (including cell lines capable of trans-complementation of replication deficient virus stocks), cell culture, and the like. Generally enzymatic reactions and purification steps are performed according to the manufacturer's specifications. The techniques and procedures are generally performed according to conventional methods in the art and various general references (see, generally, Sambrook et al. Molecular Cloning: A Laboratory Manual, 2d ed. (1989) Cold Spring Harbor Laboratory Press, Cold Spring Harbor, N.Y.; Virology, Second edition, eds. Fields BN and Knipe DM, (1990) Raven Press, New York, NY which are provided throughout this document. The procedures therein are believed to be well known in the art and are provided for the convenience of the reader.

Neoplasia is a pathological condition which is characterized, in part, by the generation of neoplastic cells having variant genotypes and phenotypes. Some tumors may comprise a population of cells lacking *RB* function but having *p53* function; such cells are designated RB⁽⁻⁾. Some tumor cells may lack *p53* function but have *RB* function; such cells are designated p53⁽⁻⁾. Some tumors may comprise cells lacking both *p53* and *RB* and are designated p53⁽⁻⁾RB⁽⁻⁾. The cell line SAOS2 (*infra*, see Experimental Example) is an example of a neoplastic cell type which is p53⁽⁻⁾RB⁽⁻⁾. Also, there may be neoplastic cells which comprise essentially normal levels of *p53* and *RB*; such cells having both normal *p53* and normal *RB* may lack other oncoproteins (e.g., tumor suppressor gene products other than *p53* or *RB*) which can provide the basis for antineoplastic viral constructs which can preferentially manifest a replication phenotype in such neoplastic cells,

A basis of the present invention is that several DNA viruses which infect mammalian cells (e.g., adenoviruses; papovaviruses such as BK and JC, SV40, and papillomaviruses such as HPV, and the like) encode viral proteins which are essential for efficient progression through the viral replication cycle; some of these viral proteins sequester cellular proteins, such as those involved in cell-cycle control and/or formation of transcription complexes, as a necessary condition for efficient viral replication. In the absence of the viral proteins which bind, sequester, or degrade such cellular proteins as *p53* and *RB*, viral replication is substantially inhibited. Normal (i.e., non-neoplastic) cells which are infected with a mutant virus lacking the ability to sequester or degrade *p53* and/or *RB* are generally unable to support replication of the mutant virus, hence such mutant viruses are considered to be replication deficient (or replication defective). However, since the sequestration or degradation of *p53* or *RB* is not necessary for viral replication in cells which lack functional *p53* or *RB* (such cells are designated p53⁽⁻⁾ and RB⁽⁻⁾ respectively) it is possible that replication deficient mutant viruses which are defective for *p53* and/or *RB* sequestration or degradation may express a replication phenotype in such p53⁽⁻⁾ or RB⁽⁻⁾ cells to a greater extent than in cells having essentially normal *p53* and/or *RB* function. Neoplastic cells frequently lack *p53* function (a p53⁽⁻⁾ cell), *RB* function (a RB⁽⁻⁾ cell), or both functions (a p53⁽⁻⁾RB⁽⁻⁾ cell). Hence, some replication deficient viral mutants may preferentially exhibit a replication phenotype in neoplastic cells.

Viral mutants lacking the capacity to express a functional *RB* inactivating protein (e.g., adenovirus E1a, HPV E7 protein) will manifest a replication phenotype in RB⁽⁻⁾ cells and RB⁽⁻⁾p53⁽⁻⁾ cells. Viral mutants lacking the capacity to express a functional *p53* inactivating protein (e.g., adenovirus E1b p55, HPV E6 protein) will manifest a replication phenotype in p53⁽⁻⁾ cells and RB⁽⁻⁾p53⁽⁻⁾ cells. Viral mutants lacking the capacity to express both a functional *p53* inactivating protein (e.g., adenovirus E1b p55, HPV E6 protein) and a functional *RB* inactivating protein (e.g., adenovirus E1a, HPV E7 protein) will manifest a replication phenotype in RB⁽⁻⁾p53⁽⁻⁾ cells. Cytotoxicity linked to the expression of a replicative phenotype can therefore be used as a basis for preferentially killing neoplastic cells having a RB⁽⁻⁾, p53⁽⁻⁾, or RB⁽⁻⁾p53⁽⁻⁾ phenotype. Although some replicating non-neoplastic cells may transiently exhibit a RB⁽⁻⁾ phenotype, p53⁽⁻⁾ phenotype, or RB⁽⁻⁾p53⁽⁻⁾ phenotype during progression through the cell cycle, the viral mutants of the invention may be used for preferential, albeit not necessarily completely selective, killing of neoplastic cells, thus constituting a useful antineoplastic therapy modality to be used alone or in combination with other modalities of treatment.

For a general description of adenovirus biology, Virology, Second edition, eds. Fields BN and Knipe DM, Vol.2, pp. 1651-1740, Raven Press, New York, NY, may be referred to for guidance. The following specific descriptions refer to, but are not limited to, adenovirus serotype 5 and adenovirus serotype 2. Although it is believed that other adenoviral serotypes may be used, adenovirus type 5 provides a common reference point for the nucleotide numbering convention of viral polynucleotides and amino acid numbering of viral-encoded polypeptides of the E1a viral gene region, and other viral genes. Adenovirus type 2 provides a convenient reference for the numbering convention of the E1b viral gene region, and other viral gene regions. It is believed that those of skill in the art will readily identify the corresponding positions in other adenoviral serotypes.

### E1b Mutants

A function of the cellular phosphoprotein *p53* is to inhibit the progression of mammalian cells through the cell cycle. Wild-type adenovirus E1b p55 protein binds to *p53* in infected cells that have *p53* and produce a substantial inactivation of *p53* function, likely by sequestering *p53* in an inactive form. Functional E1b p55 protein is essential for efficient adenoviral replication in cells containing functional *p53.* Hence, adenovirus variants which substantially lack the ability to bind *p53* are replication deficient in non-replicating, non-neoplastic cells having normal levels of functional *p53*.

Human tumor cells frequently are homozygous or heterozygous for mutated (e.g., substitution, deletion, frameshift mutants) *p53* alleles, and lack *p53* function necessary for normal control of the cell cycle (Hollstein et al. (1991) Science 253: 49; Levine et al. (1991) op.cit.). Thus, many neoplastic cells are p53⁽⁻⁾, either because they lack sufficient levels of *p53* protein and/or because they express mutant forms of *p53* which are incapable of substantial *p53* function, and which may substantially diminish *p53* function even when wild-type *p53* may be present (e.g., by inhibiting formation of functional multimers). Some neoplastic cells may comprise alleles encoding essentially wild-type *p53* proteins, but may comprise a second site mutation that substantially abrogates *p53* function, such as a mutation that results in *p53* protein being localized in the cytoplasm rather than in the nucleus; such second site mutants also substantially lack *p53* function.

It is believed that replication deficient adenovirus species which lack the capacity to complex *p53* but substantially retain other essential viral replicative functions will exhibit a replication phenotype in cells which are deficient in *p53* function (e.g., cells which are homozygous for substantially deleted *p53* alleles, cells which comprise mutant *p53* proteins which are essentially nonfunctional) but will not substantially exhibit a replicative phenotype in non-replicating, non-neoplastic cells. Such replication deficient adenovirus species are referred to herein for convenience as E1b-p53⁽⁻⁾ replication deficient adenoviruses.

A cell population (such as a mixed cell culture or a human cancer patient) which comprises a subpopulation of neoplastic cells lacking *p53* function and a subpopulation of non-neoplastic cells which express essentially normal *p53* function can be contacted under infective conditions (i.e., conditions suitable for adenoviral infection of the cell population, typically physiological conditions) with a composition comprising an infectious dosage of a E1b-p53⁽⁻⁾ replication deficient adenovirus. Such contacting results in infection of the cell population with the E1b-p53⁽⁻⁾ replication deficient adenovirus. The infection produces preferential expression of a replication phenotype in a significant fraction of the cells comprising the subpopulation of neoplastic cells lacking *p53* function but does not produce a substantial expression of a replicative phenotype in the subpopulation of non-neoplastic cells having essentially normal *p53* function. The expression of a replication phenotype in an infected p53⁽⁻⁾ cell results in the death of the cell, such as by cytopathic effect (CPE), cell lysis, apoptosis, and the like, resulting in a selective ablation of neoplastic p53⁽⁻⁾ cells from the cell population.

Typically, E1b-p53⁽⁻⁾ replication deficient adenovirus constructs suitable for selective killing of p53*(-)* neoplastic cells comprise mutations (e.g., deletions, substitutions, frameshifts) which inactivate the ability of the E1b p55 polypeptide to bind *p53* protein effectively. Such inactivating mutations typically occur in the regions of p55 which bind *p53*. Optionally, the mutant E1b region may encode and express a functional p19 protein encoded by the E1b region remains and that is functional in transactivation of adenoviral early genes in the absence of E1a polypeptides.

Suitable E1b-p53⁽⁻⁾ replication deficient adenovirus constructs for use in the methods and compositions of the invention include, but are not limited to the following examples: (1) adenovirus type 2 dl 1520, which contains a C to T mutation at nucleotide position 2022 that generates a stop codon 3 amino acids downstream of the AUG codon used for initiation of translation of the p55 protein and a deletion between nucleotides 2496 and 3323 replaced with a small linker insertion that generates a second stop codon at nucleotide 3336; the expression of the p19 protein is essentially unaffected (Barker and Berk (1987) Virology 156: 107, and (2) a composite adenovirus construct comprising adenovirus type 2 dl 1520 comprising at least the position 2022 mutation and/or the 2496-3323 deletion mutation, or a substantial portion thereof, and an additional mutation in p19 to yield a p19 *cyt* mutant; the composite virus construct lacks p55 and comprises the enhanced cytopathic effect of the p19 *cyt* mutation. Ad2 dl 1520 are available from Dr. A. Berk, University of California at Los Angeles, Los Angeles, CA, and are described in the literature, including Barker and Berk (1987) Virology 156: 107).

It may be preferable to incorporate additional mutations into such adenovirus constructs to inhibit formation of infectious virions in neoplastic cells which otherwise would support replication of the E1b-p53⁽⁻⁾ mutants. Such additional inactivating mutations would be preferred in therapeutic modalities wherein complete viral replication forming infectious virions capable of spreading to and infecting adjacent cells is undesirable. These fully inactivated mutants are referred to as nonreplicable E1b-p53⁽⁻⁾ mutants. Such nonreplicable mutants comprise mutations which prevent formation of infectious virions even in p53⁽⁻⁾RB⁽⁻⁾ cells; such mutations typically are structural mutations in an essential virion protein or protease.

However, in many modalities it is desirable for the mutant virus to be replicable and to form infectious virions containing the mutant viral genome which may spread and infect other cells, thus amplifying the antineoplastic action of an initial dosage of mutant virus.

Additional E1b⁽⁻⁾ mutants lacking the capacity to bind *p53* can be generated by those of skill in the art by generating mutations in the E1b gene region encoding the p55 polypeptide, expressing mutant p55 polypeptides, contacting the mutant p55 polypeptides with *p53* or a binding fragment of *p53* under aqueous binding conditions, and identifying mutant E1b polypeptides which do not specifically bind *p53* as being candidate E1b⁽⁻⁾ mutants suitable for use in the invention.

More typically, a functional assay will be used to identify candidate E1b⁽⁻⁾ mutants. For example, the Friend assay for determination of *p53* function will be performed essentially as described in Frebourg et al. (1992) Cancer Res. 52: 6977. E1b mutants which lack the capacity to inactivate *p53* will be identified as candidate E1b⁽⁻⁾ replication deficient mutants.

### E1a/E1b Double Mutants

The loss of retinoblastoma tumor suppressor gene (*RB*) gene function has been associated with the etiology of various types of tumors. The product of this tumor suppressor gene, a 105 kilodalton polypeptide called pRB or p105, is a cell-cycle regulatory protein. The pRB polypeptide inhibits cell proliferation by arresting cells at the G₁ phase of the cell cycle. The pRB protein is also a major target of several DNA virus oncoproteins, including adenovirus E1a, SV40 large T Ag, and papillomavirus E7. These viral proteins bind and inactivate pRB, and the function of inactivating pRB is important in facilitating viral replication. The pRB protein interacts with the E2F transcription factor, which is involved in the expression of the adenovirus E2 gene and several cellular genes, and inhibits the activity of this transcription factor (Bagchi et al. (1991) Cell 65: 1063; Bandara et al. (1991) Nature 351: 494; Chellappan et al. (1992) Proc. Natl. Acad. Sci. (U.S.A.) 89: 4549). The viral oncoproteins, such as adenovirus E1a, disrupt the pRB/E2F complex resulting in activation of E2F.

Some human tumor cells lack both *p53* function and *RB* function, either by mutational inactivation or deletion of one or both protein species. Such cells are termed p53⁽⁻⁾RB⁽⁻⁾ cells.

It is believed that replication deficient adenovirus species which lack the capacity to bind *p53* and which also lack the capacity to bind *RB*, but which substantially retain other essential viral replicative functions will preferentially exhibit a replication phenotype in p53⁽⁻⁾RB⁽⁻⁾ cells. Such replication deficient adenovirus species are referred to herein for convenience as E1a-RB⁽⁻⁾/E1b-p53⁽⁻⁾ replication deficient adenoviruses, or simply E1a/E1b double mutants. Such E1a/E1b double mutants can be constructed by those of skill in the art by combining at least one E1a-RB⁽⁻⁾ mutation in the E1a region and at least one E1b-p53⁽⁻⁾ mutation in E1b region encoding p55 to form a E1a/E1b double mutant adenovirus. Such a replication deficient double mutant adenovirus will exhibit a replication phenotype in cells which are deficient in both *p53* and *RB* functions but will not substantially exhibit a replicative phenotype in non-replicating, non-transformed cells or in cells which are deficient in either *p53* or *RB* function but not both functions. For example, the Ad5 dl 434 mutant (Grodzicker et al. (1980) Cell 21: 454) comprises a deletion of the E1a locus and a partial deletion of the E1b locus, and substantially lacks the capacity to encode functional E1a and E1b p55 proteins.

Typically, E1a-RB⁽⁻⁾ replication deficient adenovirus constructs suitable for selective killing of RB*(-)* neoplastic cells comprise mutations (e.g., deletions, substitutions, frameshifts) which inactivate the ability of an E1a polypeptide to bind *RB* protein effectively. Such inactivating mutations typically occur in the E1a CR1 domain (amino acids 30-85 in Ad5: nucleotide positions 697-790) and/or the CR2 domain (amino acids 120-139 in Ad5; nucleotide positions 920-967), which are involved in binding the p105 *RB* protein and the p107 protein. Preferably, the CR3 domain (spanning amino acids 150-186) remains and is expressed as a truncated p289R polypeptide and is functional in transactivation of adenoviral early genes. Fig. 1 portrays schematically the domain structure of the E1a-289R polypeptide.

Suitable E1a-RB⁽⁻⁾ replication deficient adenovirus constructs include, but are not limited to the following examples: (1) adenovirus serotype 5 NT dl 1010, which encodes an E1a protein lacking the CR1 and CR2 domains (deletion of amino acids 2 to 150; nucleotides 560-1009) necessary for efficient *RB* binding, but substantially retaining the CR3 domain (Whyte et al. (1989) Cell 56: 67), and (2) adenovirus serotype 5 dl 312, which comprises a deleted viral genome lacking the region spanning nucleotides 448-1349 which encodes the entire E1a region in wild-type adenovirus (Jones N and Shenk T (1979) Proc. Natl. Acad. Sci. (U.S.A.) 76: 3665). Ad5 NT dl 1010 is a preferred E1a-RB⁽⁻⁾ replication deficient adenovirus and is available from Dr. E. Harlow, Massachusetts General Hospital, Boston, MA).

It may be preferable to incorporate additional mutations into such adenovirus constructs to inhibit formation of infectious virions in neoplastic cells which otherwise would support replication of the E1a-RB⁽⁻⁾ mutants. Such additional inactivating mutations would be preferred in therapeutic modalities wherein complete viral replication forming infectious virions capable of spreading to and infecting adjacent cells is undesirable. These fully inactivated mutants are referred to as nonreplicable E1a-RB⁽⁻⁾ mutants. Such nonreplicable mutants comprise mutations which prevent formation of infectious virions even in p53⁽⁻⁾RB⁽⁻⁾ cells; such mutations typically are structural mutations in an essential virion protein or protease.

However, in many modalities it is desirable for the mutant virus to be replicable and to form infectious virions containing the mutant viral genome which may spread and infect other cells, thus amplifying the antineoplastic action of an initial dosage of mutant virus.

Additional E1a⁽⁻⁾ mutants lacking the capacity to bind *RB* can be generated by those of skill in the art by generating mutations in the E1a gene region encoding E1a polypeptides, typically in the CR1 and/or CR2 domains, expressing the mutant E1a polypeptide, contacting the mutant E1a polypeptides with p105 or a binding fragment of *RB* under aqueous binding conditions, and identifying mutant E1a polypeptides which do not specifically bind *RB* as being candidate E1a⁽⁻⁾ mutants suitable for use in the invention. Alternative assays include contacting the mutant E1a polypeptides with the 300kD protein and/or p107 protein or binding fragment thereof under aqueous binding conditions, and identifying mutant E1a polypeptides which do not specifically bind the 300kD and/or p107 polypeptides as being candidate E1a⁽⁻⁾ mutants suitable for use in the invention. Alternative binding assays include determining the inability of E1a⁽⁻⁾ mutant protein (or absence of E1a protein) to form complexes with the transcription factor E2F and/or to lack the ability to dissociate the *RB* protein from *RB*:E2F complexes under physiological conditions (Chellappan et al. (1991) op.cit.).

Alternative functional assays for determining mutants lacking E1a function, such as loss of transctivation by E1a of transcription of various reporter polypeptides linked to a E1a-dependent transcriptional regulatory sequence, and the like, will be used.

A cell population (such as a mixed cell culture or a human cancer patient) which comprises a subpopulation of neoplastic cells lacking *p53* and *RB* functions and a subpopulation of non-neoplastic cells which express essentially normal *p53* function and/or *RB* function can be contacted under infective conditions (i.e., conditions suitable for adenoviral infection of the cell populations typically physiological conditions) with a composition comprising an infectious dosage of a replication deficient E1a/E1b double mutant adenovirus. Such contacting results in infection of the cell population with the E1a/E1b double mutant replication deficient adenovirus. The infection produces preferential expression of a replication phenotype in a significant fraction of the cells comprising the subpopulation of neoplastic cells lacking both *p53* function and *RB* function but does not produce a substantial expression of a replicative phenotype in the subpopulation of non-neoplastic cells having essentially normal *p53* function and/or *RB* function. The expression of a replication phenotype in an infected p53⁽⁻⁾RB⁽⁻⁾ cell results in the death of the cell, such as by cytopathic effect (CPE), cell lysis, and the like, resulting in a selective ablation of neoplastic p53⁽⁻⁾RB⁽⁻⁾ cells from the cell population.

It may be preferable to incorporate additional mutations into such adenovirus constructs to inhibit formation of infectious virions in neoplastic cells which otherwise would support replication of an E1a/E1b double mutant. Such additional inactivating mutations would be preferred in therapeutic modalities wherein complete viral replication forming infectious virions capable of spreading to and infecting adjacent cells is undesirable. These fully inactivated mutants are referred to as nonreplicable E1a/ E1b double mutants. Such nonreplicable mutants comprise mutations which prevent formation of infectious virions even in p53⁽⁻⁾RB⁽⁻⁾ cells; such mutations typically are structural mutations in an essential virion protein or protease.

However, in many modalities it is desirable for the mutant virus to be replicable and to form infectious virions containing the mutant viral genome which may spread and infect other cells, thus amplifying the antineoplastic action of an initial dosage of mutant virus.

### Negative Selection Viral Constructs

Although expression of an adenoviral replication phenotype in an infected cell correlates with viral-induced cytotoxicity, generally by cell lysis, cytopathic effect (CPE), apoptosis, or other mechanisms of cell death, it may often be preferable to augment the cytotoxicity of a recombinant adenovirus that `s to be used for antineoplastic therapy. Such augmentation may take the form of including a negative selection gene in the recombinant adenovirus, typically operably linked to an adenoviral promoter which exhibits positive transcriptional modulation in cells expressing a replication phenotype. For example, a HSV *tk* gene cassette may be operably linked immediately downstream of an E3 promoter of a replication deficient adenovirus, such as Ad5 NT dl 1110. Frequently, it is desirable to delete a nonessential portion (i.e., for viral replication and packaging) of the adenoviral genome to accommodate the negative selection cassette; thus a substantial portion of the E3 gene region may be deleted and replaced with a negative selection cassette such as an HSV *tk* gene operably linked to an E2 promoter (and enhancer) or other suitable promoter/enhancer. Alternatively, a negative selection gene may be operably linked to an adenovirus late region promoter to afford efficient expression of the negative selection gene product in cells expressing a replication phenotype characterized by transcription from late gene promoters.

For embodiments where viral replication forming infectious virions in vivo is undesirable, adenovirus replication deficient constructs which are nonreplicable are used. Such nonreplicable mutants comprise an E1a⁽⁻⁾ and/or E1b⁽⁻⁾ mutation and comprise all genetic functions necessary for generating a replication phenotype in a suitable neoplastic cell (e.g., a p53⁽⁻⁾ cell, a RB⁽⁻⁾ cell, or a p53⁽⁻⁾RB⁽⁻⁾ cell) but have deleted at least one essential gene function necessary for formation of infectious virions, such as structural coat proteins, proteases, and the like. Alternatively, an elicited immune response evoked by the virus may neutralize infectious virions and moderate spread of the viral infection. Nonreplicable mutants lacking a complementable trans-acting function in addition to an E1a and/or E1b mutation may be propagated in conjunction with a complementary helper virus or a helper cell line capable of providing the deleted trans-acting function(s). For example, the 293 cell line (ATCC # CRL 1573; Graham et al. (1977) J. Gen. Virol. 36: 59) which provides E1a and E1b functions in trans may be modified to provide additional functions in trans, such as a virion coat protein or the like, to permit propagation of the "nonreplicable" mutants for developing virus stocks.

Expression of the HSV *tk* gene in a cell is not directly toxic to the cell, unless the cell is exposed to a negative selection agent such as gancyclovir or FIAU. Infected cells expressing a replication phenotype wherein a negative selection gene is substantially expressed may produce essentially no additional cytotoxicity until the negative selection agent (e.g., gancyclovir) is administered in an effective selective dosage, at which time the infected cells expressing the *tk* gene will be selectively ablated; thus negative selection can be used for enhanced cytopathic killing and/or to damp out further viral replication by killing cells exhibiting a replicative phenotype. Further, by adjusting the dosages and/or administration schedule of the negative selection agent, it is possible to produce only a partial ablation of the infected cell population expressing the negative selection gene. Generally, the dosage of gancyclovir is calibrated by generating a standard dose-response curve and determining the dosage level at which a desired level of ablation of infected neoplastic cells is observed. Information regarding administration of gancyclovir (GANC) to animals is available in various sources in the art, including human prescribing directions from package inserts. When used in cell culture, a selective concentration of gancyclovir is typically in the range of 0.05µM to 50 µM, typically about 1 µM, with about 0.2 µM used for in vitro applications and about 1-5 µM administered for in vivo applications (typically administered over about 24 hours by continuous infusion from an osmotic pump loaded with 125mg/ml of gancyclovir in aqueous solution). A dosage schedule for in vivo administration may comprise gancyclovir at a dosage of 5 mg/kg bodyweight B.I.D., given intravenously for seven days.

Negative selection genes may be incorporated into E1a-RB⁽⁻⁾ replication deficient adenovirus constructs, E1ba-p53⁽⁻⁾ replication deficient adenoviral constructs, E1a/E1b double mutant replication deficient viral constructs, or the like. A preferred embodiment is an HSV *tk* gene cassette (Zjilstra et al. (1989) Nature 342:435; Mansour et al. (1988) Nature 336: 348; Johnson et al. (1989) Science 245: 1234: Adair et al. (1989) Proc. Natl. Acad. Sci (U.S.A.) 86: 4574; Capecchi, M. (1989) Science 244:1288) operably linked to the E2 promoter of Ad5 NT dl1110 or an alternative promoter and/or enhancer (e.g., major late promoter, E1a promoter/enhancer, E1b promoter/enhancer), with a polydenylation site to form a *tk* expression cassette. The *tk* expression cassette (or other negative selection expression cassette) is inserted into the adenoviral genome, for example, as a replacement for a substantial deletion of the E3 gene. Other negative selection genes and replication deficient adenovirus constructs will be apparent to those of skill in the art. It is believed that a negative selection gene operably linked to the E2 promoter is an especially preferred embodiment for incorporation into E1a⁽⁻⁾ replication-deficient adenovirus mutants, as the E2 promoter contains multiple E2F sites, whereas RB⁽⁻⁾ and p53⁽⁻⁾RB⁽⁻⁾ lack *RB* function and presumably will exhibit more efficient transcription from the E2 promoter.

### Diagnostic and In Vitro Uses

The replication deficient adenoviruses of the invention may be used to detect the presence of cells lacking *p53* and optionally also *RB* function. For example, a cell sample comprising a subpopulation of neoplastic cells lacking *p53* and optionally also *RB* can be infected with a suitable replication deficient adenovirus species. After a suitable incubation period, the cells in the cell sample that express a replication phenotype (e.g., loss of ability to exclude Trypan blue, virion formation, ³H-thymidine incorporation into viral DNA) can be quantified to provide a measure of the number or proportion of replicative and/or neoplastic cells in the cell sample. Such methods may be used to diagnose neoplasms and/or evaluate tumor cell load following chemotherapy in a patient on the basis of an explanted cell sample (e.g., a lymphocyte sample from a patient undergoing chemotherapy for a lymphocytic leukemia).

Alternative diagnostic uses and variations are apparent; for example, a reporter gene (e.g., luciferase, β-galactosidase) may be substituted for a negative selection gene in a replication deficient adenovirus; transformed cells may be scored (such as in a cellular sample or transformation assay) by the expression of the reporter gene, which is correlated with expression of a replication phenotype indicating a lack of *p53* and optionally also *RB* in a cell.

### Therapeutic Methods

Therapy of neoplastic disease may be afforded by administering to a patient a composition comprising replication defective adenoviruses of the invention, including: E1b-p53⁽⁻⁾ replication deficient adenoviruses, E1a/E1b double mutants, and replication deficient adenoviruses further comprising a negative selection gene.

Various human neoplasms comprising cells that lack *p53* and optionally also *RB* functions may be treated with the replication deficient adenoviral constructs. For example but not limitation, a human patient or nonhuman mammal having a bronchogenic carcinoma, nasopharyngeal carcinoma, laryngeal carcinoma, small cell and non-small cell lung carcinoma, lung adenocarcinoma, hepatocarcinoma, pancreatic carcinoma, bladder carcinoma, colon carcinoma, breast carcinoma, cervical carcinoma, ovarian carcinoma, or lymphocytic leukemias may be treated by administering an effective antineoplastic dosage of an appropriate replication deficient adenovirus. Suspensions of infectious adenovirus particles may be applied to neoplastic tissue by various routes, including intravenous, intraperitoneal, intramuscular, subdermal, and topical. A adenovirus suspension containing about 10³ to 10¹² or more virion particles per ml may be inhaled as a mist (e.g., for pulmonary delivery to treat bronchogenic carcinoma, small-cell lung carcinoma, non-small cell lung carcinoma, lung adenocarcinoma, or laryngeal cancer) or swabbed directly on a tumor site for treating a tumor (e.g., bronchogenic carcinoma, nasopharyngeal carcinoma, laryngeal carcinoma, cervical carcinoma) or may be administered by infusion (e.g., into the peritoneal cavity for treating ovarian cancer, into the portal vein for treating hepatocarcinoma or liver metastases from other non-hepatic primary tumors) or other suitable route, including direct injection into a tumor mass (e.g., a breast tumor), enema (e.g., colon cancer), or catheter (e.g., bladder cancer).

Candidate antineoplastic adenovirus mutants may be further evaluated by their capacity to reduce tumorigenesis or neoplastic cell burden in nu/nu mice harboring a transplant of neoplastic cells lacking *p53* and optionally also *RB* function, as compared to untreated mice harboring an equivalent transplant of the neoplastic cells.

Antineoplastic replication deficient adenovirus mutants may be formulated for therapeutic and diagnostic administration to a patient having a neoplastic disease. For therapeutic or prophylactic uses, a sterile composition containing a pharmacologically effective dosage of one or more species of antineoplastic replication deficient adenovirus mutant is administered to a human patient or veterinary non-human patient for treatment of a neoplastic condition. Generally, the composition will comprise about 10³ to 10¹⁵ or more adenovirus particles in an aqueous suspension. A pharmaceutically acceptable carrier or excipient is often employed in such sterile compositions. A variety of aqueous solutions can be used, e.g., water, buffered water, 0.4% saline, 0.3% glycine and the like. These solutions are sterile and generally free of particulate matter other than the desired adenoviral virions. The compositions may contain pharmaceutically acceptable auxiliary substances as required to approximate physiological conditions such as pH adjusting and buffering agents, toxicity adjusting agents and the like, for example sodium acetate, sodium chloride, potassium chloride, calcium chloride, sodium lactate, etc. Excipients which enhance infection of cells by adenovirus may be included.

Replication deficient viruses may be delivered to neoplastic cells by liposome or immunoliposome delivery; such delivery may be selectively targeted to neoplastic cells on the basis of a cell surface property present on the neoplastic cell population (e.g., the presence of a cell surface protein which binds an immunoglobulin in an immunoliposome). Typically, an aqueous suspension containing the virions are encapsulated in liposomes or immunoliposomes. For example, a suspension of replication deficient adenovirus virions can be encapsulated in micelles to form immunoliposomes by conventional methods (U.S. Patent 5,043,164, U.S. Patent 4,957,735, U.S. Patent 4,925,661; Connor and Huang (1985) J. Cell Biol. 101: 582; Lasic DD (1992) Nature 355: 279; Novel Drug Delivery (eds. Prescott LF and Nimmo WS: Wiley, New York, 1989); Reddy et al. (1992) J. Immunol. 148: 1585). Immunoliposomes comprising an antibody that binds specifically to a cancer cell antigen (e.g., CALLA, CEA) present on the cancer cells of the individual may be used to target virions to those cells.

The compositions containing the present antineoplastic replication deficient adenoviruses or cocktails thereof can be administered for prophylactic and/or therapeutic treatments of neoplastic disease. In therapeutic application, compositions are administered to a patient already affected by the particular neoplastic disease, in an amount sufficient to cure or at least partially arrest the condition and its complications. An amount adequate to accomplish this is defined as a "therapeutically effective dose" or "efficacious dose." Amounts effective for this use will depend upon the severity of the condition, the general state of the patient, and the route of administration.

In prophylactic applications, compositions containing the antineoplastic replication deficient adenoviruses or cocktails thereof are administered to a patient not presently in a neoplastic disease state to enhance the patient's resistance to recurrence of a neoplasm or to prolong remission time. Such an amount is defined to be a "prophylactically effective dose." In this use, the precise amounts again depend upon the patient's state of health and general level of immunity.

Single or multiple administrations of the compositions can be carried out with dose levels and pattern being selected by the treating physician. In any event, the pharmaceutical formulations should provide a quantity of the antineoplastic replication deficient adenoviruses of this invention sufficient to effectively treat the patient.

Antineoplastic replication deficient adenoviral therapy of the present invention may be combined with other antineoplastic protocols, such as conventional chemotherapy.

### Propagation of Mutant Adenovirus

Adenoviral mutants of the invention (e.g., E1a-RB⁽⁻⁾ replication deficient adenoviruses, E1b-p53⁽⁻⁾ replication deficient adenoviruses, and E1a/E1b double mutants) typically are propagated as viral stocks in a cell line (e.g., the 293 cell line ATCC # CRL 1573, American Type Culture Collection, Rockville, MD; Graham et al. (1977) J. Gen. Virol. 36: 59) which can provide E1a function, E1b function, or both E1a and E1b functions, respectively, in trans to support replication and formation of infectious mutant virions.

The following examples are offered by way of example and not by way of limitation. Variations and alternate embodiments will be apparent to those of skill in the art.

### EXPERIMENTAL EXAMPLE

The following experimental example demonstrates that administering a replication-deficient recombinant adenovirus preparation to neoplastic cells lacking *p53* and/or *Rb* function can effectively kill neoplastic cells. The example also shows that non-neoplastic cells containing *p53* and *Rb* function are relatively resistant to killing by the replication-deficient recombinant adenovirus preparation. Therefore, the data presented hereinbelow provide experimental verification that administration of replication-deficient recombinant adenovirus can be used to selectively kill neoplastic cells. The selective killing is provided by exploiting the differential ability of the mutant adenoviruses to induce a replication phenotype in neoplastic cells, but substantially not induce a replication phenotype (or associated cytopathic effect) in non-neoplastic cells.

Control non-neoplastic cells and cell lines representing a variety of neoplastic cell types were plated in 6-well culture dishes at or near confluence in DMEM (high glucose) with 10% fetal bovine serum and incubated at 37°C, 5% CO₂ under standard culturing conditions. Cells were plated to be screened at a density of 5 x 10⁵ cells/well. The neoplastic cell lines tested were: SAOS-2 (ATCC HTB85), derived from a human primary osteogenic sarcoma; U-2OS (ATCC HTB96), derived from a human osteogenic sarcoma; HS700T, (ATCC HTB147), derived from a human metastatic adenocarcinoma originating in the pancreas or intestines; 293 (ATCC CRL1573), transformed human embryonal kidney; and DLD-1 (ATCC CCL221), derived from a human colon adenocarcinoma. Each of the cell lines is available from American Type Culture Collection, Rockville, MD. The control non-neoplastic cells were IMR90 (ATCC CCL 186) and WI-38 (ATCC CCL 75), both diploid human lung fibroblast lines.

These cell cultures were subsequently infected, in parallel, with wild-type adenovirus Type 2, and replication-deficient recombinant adenovirus mutants dl 11010, dl 434, and dl 1520. An extra culture dish was plated for the purpose of counting cells. These cells came from the same suspension of cells used for the viral infections. Cells were counted in order to determine the number of viral plaque-forming units (PFU) to add to the cell cultures for a desired multiplicity of infection (MOI). The wild-type adenovirus Type 2 and the mutant adenoviruses were added to the parallel cell cultures at MOIs of 0.1, 1.0, 10, and 100. Virus suspended in PBS was added to the cell wells in a volume of 1 ml. The inoculated culture dishes were rocked in both the X- and Y- axes immediately after inoculation and halfway through the adsorption time of approximately one hour at 37°C, 5% CO₂. After the one hour adsorption period, 2 ml of DMEM with high glucose and 2% fetal bovine serum was added, and the cultures incubated at 37°C, 5% CO₂ under standard culturing conditions.

At various times after infection, see, Fig. 2(a-i), cell cultures were stained with 0.5% crystal violet in 20% methanol in order to determine the efficacy of cell killing by the virus preparations. Dead cells were detached and rinsed out of the wells, whereas living cells remained in the well and were stained with the dye. The results demonstrate that the replication-deficient recombinant adenovirus preparations were able to preferentially kill neoplastic cells as compared to non-neoplastic cells, and that wild-type adenovirus Type 2 killed both neoplastic and non-neoplastic cells approximately equally well. The results demonstrate that the dl 11010 mutant was particularly effective at killing neoplastic cells, with the dl 1520 mutant and the dl 434 mutant also being effective. An example of the results is shown in Fig. 2a-i, which are photographs of the stained cell wells obtained at various times after infection with wild-type adenovirus 2, the mutants: dl 1010 (E1A⁽⁻⁾), dl1520 (E1B⁽⁻⁾), or dl 434. Fig. 2a shows that in the absence of virus, each of the cell cultures had grown to confluence and were attached to the culture wells. Fig. 2b-c shows that wild-type adenovirus 2 was able to kill cells in each of the culture wells, although with variable efficacy and incompletely, with at least some cells staining in each well. Note that WI-38 and SAOS-2 lines are not good hosts for adenovirus infection and that IMR90 (Fig. 3a-c) serves as an alternate control cell line for human diploid fibroblasts. Fig. 2d-e shows that the dl 1010 mutant was able to kill effectively all of the neoplastic cell lines except the infection-resistant SAOS-2. dl 1010 killed the 293, U2OS, HS700T, and DLD-1 lines by 12 days after infection and did not substantially kill diploid human lung fibroblasts (WI-38). Fig. 2f-g shows that the dl 1520 mutant was able to kill effectively 3 of the 5 neoplastic cell lines (293, HS700T, and DLD-1) by 14-20 days after infection and did not substantially kill diploid human lung fibroblasts (WI-38). dl 1520 is an E1B⁽⁻⁾ mutant, and cell line U2OS does not allow such an E1B⁽⁻⁾ mutant virus to replicate, indicating specificity of the different transformed cell lines for infection by the mutant recombination-defective adenoviruses, as predicted. Fig. 2h-i shows that the dl 434 mutant (a double-mutant: E1A⁽⁻⁾E1B⁽⁻⁾) was able to kill effectively 3 of the 5 neoplastic cell lines (293, HS700T, and DLD-1) by 14-20 days after infection and did not substantially kill diploid human lung fibroblasts (WI-38). The DLD-1 and U2OS lines displayed a partially resistant phenotype for replication of dl 434.

Fig. 3(a-c) show that IMR90 human diploid lung fibroblasts were killed differentially by wild-type Ad2, dl 1010, and dl 1520. The 293 cell line serves as a positive control. Fig. 3(a) shows that wild-type Ad2 killed the IMR90 cells effectively at an MOI of 10 or 100. Fig. 3(b) shows that dl 1010 was unable to replicate in IMR90 cells at the highest MOI tested, even though dl 1010 killed neoplastic cell lines 293, U2OS, HS700T, and DLD-1 as shown in Fig. 2(d-e). Fig. 3(c) shows that dl 1520 virus was able to kill IMR90 cells effectively only at an MOI of 100; at this high dosage of virus the possibility that the cells die as a result of virus overload rather than replication cannot be ruled out.

Thus, the data shown in Figs. 2(a-i) and 3(a-c) indicate that replication-deficient recombinant adenovirus mutants can be used to selectively kill neoplastic cells lacking *p53* and/or *Rb* function, as predicted.

## Claims

1. A recombinant replication deficient adenovirus lacking an expressed viral oncoprotein capable of binding a functional p53 tumour suppressor gene product, for use in a method of treatment or therapy of a mammal;
said treatment or therapy comprising the ablation of neoplastic cells in a cell population comprising said neoplastic cells and non-neoplastic cells;
said non-neoplastic cells containing a functional p53 tumour suppressor gene product capable of forming a bound complex with said viral oncoprotein and said neoplastic cells lacking said functional p53 tumour suppressor gene product.

2. A recombinant replication deficient adenovirus for use according to claim 1 lacking an expressed viral E1b oncoprotein capable of binding a functional tumour suppressor gene product.

3. A recombinant replication deficient adenovirus for use according to claim 1 or claim 2 wherein the neoplastic cells are p53⁽⁻⁾RB⁽⁻⁾.

4. A recombinant replication deficient adenovirus for use according to any one of the preceding claims wherein the recombinant replication deficient adenovirus does not encode an E1a polypeptide capable of binding RB as well as does not encode a E1b p55 polypeptide capable of binding p53.

5. A recombinant replication deficient adenovirus for use according to claim 1, wherein said virus is adenovirus type 2 dl1520 or adenovirus type 5 dl434.

6. A recombinant replication deficient adenovirus for use according to any one of the preceding claims wherein the mammal is a human.

7. A recombinant replication deficient adenovirus according to any one of the preceding claims, wherein the adenovirus is capable of forming infectious virions in the neoplastic cell which are able to spread and infect adjacent cells in vivo in a patient.

8. A recombinant replication deficient adenovirus for use according to any one of the preceding claims which is nonreplicable for forming infectious virions in non-neoplastic cells of a human patient.

9. A recombinant replication deficient adenovirus for use according to any one of the preceding claims further comprising a negative selection gene operably linked to a viral promoter.

10. A recombinant replication deficient adenovirus for use according to any one of the preceding claims, wherein said treatment or therapy further comprises contacting said cells with a chemotherapeutic.

11. A pharmaceutical composition comprising a recombinant replication deficient adenovirus as defined in any one of claims 1 to 10, and a pharmaceutically acceptable carrier.

12. A pharmaceutical composition according to claim 11 further comprising a chemotherapeutic.

13. Use of a recombinant replication deficient adenovirus lacking an expressed viral oncoprotein capable of binding a functional p53 tumour suppressor gene product, for the manufacture of a medicament for the ablation of neoplastic cells in a cell population comprising said neoplastic cells and non-neoplastic cells;
said non-neoplastic cells containing a functional p53 tumour suppressor gene product capable of forming a bound complex with said viral oncoprotein and said neoplastic cells lacking said functional p53 tumour suppressor gene product.

14. Use according to claim 13 wherein said recombinant replication deficient adenovirus is as defined in any one of claims 2 to 10.

15. Use according to claim 13 or 14 for the treatment of a p53 negative cancer selected from a bronchogenic carcinoma, nasopharyngeal carcinoma, laryngeal carcinoma, small cell and non-small cell lung carcinoma, lung adenocarcinoma, hepatocarcinoma, pancreatic carcinoma, bladder carcinoma, colon carcinoma, breast carcinoma, cervical carcinoma, ovarian carcinoma, or lymphocytic leukemia.

16. An *in vitro* method of ablating neoplastic cells lacking a functional p53 tumour suppressor gene product in a cell population of neoplastic and non-neoplastic cells which have p53 tumour suppressor function, said method comprising:
contacting under infective conditions (1) a recombinant replication deficient adenovirus lacking an expressed viral oncoprotein capable of binding a functional p53 tumour suppressor gene product, with (2) a cell population comprising said neoplastic cells and non-neoplastic cells, thereby generating an infected cell population such that ablation of said neoplastic cells occurs.

17. A method according to claim 16 wherein said virus is as defined in any one of claims 1 to 10.

18. A method according to claim 16 or 17 wherein said neoplastic cell lacking p53 suppressor function are cancer cells from a bronchogenic carcinoma, nasopharyngeal carcinoma, laryngeal carcinoma, small cell and non-small cell lung carcinoma, lung adenocarcinoma, hepatocarcinoma, pancreatic carcinoma, bladder carcinoma, colon carcinoma, breast carcinoma, cervical carcinoma, ovarian carcinoma, or lymphocytic leukemia.

19. A method according to any one of Claims 16 to 18 wherein said method further comprises contacting said cell population with a chemotherapeutic.

20. An *in vitro* method for detecting the absence of functional tumour suppressor activity in a cell population, comprising:
contacting said cell population with a recombinant replication deficient adenovirus lacking an expressed viral oncoprotein capable of binding a functional p53 tumour suppressor gene product, under infective conditions, and determining the number or proportion of cells in said population with a replication phenotype.

21. A method according to claim 20 wherein said determining comprises measuring ability of cells to exclude dye, to incorporate a labelled nucleotide into viral DNA, or to form virions.

## Patentansprüche

1. Rekombinantes Adenovirus mit Replikationsdefizienz, dem ein exprimiertes virales Onkoprotein fehlt, das zur Bindung eines funktionellen p53-Tumorsuppressor-Genprodukts fähig ist, zur Verwendung bei einem Verfahren zur Behandlung oder Therapie eines Säugetiers;
wobei die Behandlung oder Therapie die Ablation neoplastischer Zellen in einer Zellpopulation umfaßt, welche die neoplastischen Zellen und nicht-neoplastische Zellen umfaßt;
wobei die nicht-neoplastischen Zellen ein funktionelles p53-Tumorsuppressor-Genprodukt enthalten, das zur Bildung eines gebundenen Komplexes mit dem viralen Onkoprotein fähig ist, und den neoplastischen Zellen das funktionelle p53-Tumorsuppressor-Genprodukt fehlt.

2. Rekombinantes Adenovirus mit Replikationsdefizienz zur Verwendung nach Anspruch 1, dem ein exprimiertes virales E1b-Onkoprotein fehlt, das zur Bindung eines funktionellen Tumorsuppressor-Genprodukts fähig ist.

3. Rekombinantes Adenovirus mit Replikationsdefizienz zur Verwendung nach Anspruch 1 oder 2, worin die neoplastischen Zellen p53⁽⁻⁾RB⁽⁻⁾ sind.

4. Rekombinantes Adenovirus mit Replikationsdefizienz zur Verwendung nach einem der vorangegangenen Ansprüche, worin das rekombinante Adenovirus mit Replikationsdefizienz nicht für ein E1a-Polypeptid kodiert, das zur Bindung von RB fähig ist, und ebenso nicht für ein E1b p55-Polypeptid kodiert, das zur Bindung von p53 fähig ist.

5. Rekombinantes Adenovirus mit Replikationsdefizienz zur Verwendung nach Anspruch 1, worin das Virus Adenovirus Typ 2 dl1520 oder Adenovirus Typ 5 dl434 ist.

6. Rekombinantes Adenovirus mit Replikationsdefizienz zur Verwendung nach einem der vorangegangenen Ansprüche, worin das Säugetier ein Mensch ist.

7. Rekombinantes Adenovirus mit Replikationsdefizienz nach einem der vorangegangenen Ansprüche, worin das Adenovirus zur Bildung von infektiösen Virionen in der neoplastischen Zelle fähig ist, die sich in vivo in einem Patienten auf benachbarte Zellen ausbreiten und diese infizieren können.

8. Rekombinantes Adenovirus mit Replikationsdefizienz zur Verwendung nach einem der vorangegangenen Ansprüche, das nicht-replizierbar ist, uni infektiöse Virione in nicht-neoplastischen Zellen eines Menschen zu bilden.

9. Rekombinantes Adenovirus mit Replikationsdefizienz zur Verwendung nach einem der vorangegangenen Ansprüche, das weiters ein negatives Selektionsgen umfaßt, daß mit einem viralen Promotor operabel verbunden ist.

10. Rekombinantes Adenovirus mit Replikationsdefizienz zur Verwendung nach einem der vorangegangenen Ansprüche, worin die Behandlung oder Therapie weiters das Kontaktieren der Zellen mit einem Chemotherapeutikum umfaßt.

11. Pharmazeutische Zusammensetzung, die ein rekombinantes Adenovirus mit Replikationsdefizienz, wie in einem der Ansprüche 1 bis 10 definiert, und einen pharmazeutisch annehmbaren Träger umfaßt.

12. Pharmazeutische Zusammensetzung nach Anspruch 11, die weiters ein Chemotherapeutikum umfaßt.

13. Verwendung eines rekombinanten Adenovirus mit Replikationsdefizienz, dem ein exprimiertes virales Onkoprotein fehlt, das zur Bindung eines funktionellen p53-Tumorsuppressor-Genprodukts fähig ist, zur Herstellung eines Medikaments zur Ablation neoplastischer Zellen in einer Zellpopulation, welche die neoplastischen Zellen und nicht-neoplastische Zellen enthält;
wobei die nicht-neoplastischen Zellen ein funktionelles p53-Tumorsuppressor-Genprodukt enthalten, das zur Bildung eines gebundenen Komplexes mit dem viralen Onkoprotein und den neoplastischen Zellen fähig ist, denen das funktionelle p53-Tumorsuppressor-Genprodukt fehlt.

14. Verwendung nach Anspruch 13, worin das rekombinantes Adenovirus mit Replikationsdefizienz wie nach einem der Ansprüche 2 bis 10 definiert ist.

15. Verwendung nach Anspruch 13 oder 14 zur Behandlung von p53-negativem Krebs, der aus bronchogenem Karzinom, nasopharyngealem Karzinom, Kehlkopfkarzinom, Kleinzellen- und Nicht-Kleinzellen-Lungenkarzinom, Lungenadenokarzinom, Hepatokarzinom, Pankreaskarzinom, Blasenkarzinom, Darmkarzinom, Brustkarzinom, Zervixkarzinom, Eierstockkarzinom oder lymphozytischer Leukämie ausgewählt ist.

16. In vitro-Verfahren zur Ablation neoplastischer Zellen, denen ein funktionelles p53-Tumorsuppressor-Genprodukt fehlt, in einer Zellpopulation aus neoplastischen und nicht-neoplastischen Zellen, die p53-Tumorsuppressorfunktion aufweisen, wobei das Verfahren umfaßt:
das Kontaktieren (1) eines rekombinanten Adenovirus mit Replikationsdefizienz, dem ein exprimiertes virales Onkoprotein fehlt, das zur Bindung eines funktionellen p53-Tumorsuppressor-Genprodukts fähig ist, mit (2) einer Zellpopulation, welche die neoplastischen Zellen und nicht-neoplastische Zellen umfaßt, unter infektiösen Bedingungen, wodurch eine infizierte Zellpopulation erzeugt wird, so daß Ablation der neoplastischen Zellen erfolgt.

17. Verfahren nach Anspruch 16, worin das Virus wie nach einem der Ansprüche 1 bis 10 definiert ist.

18. Verfahren nach Anspruch 16 oder 17, worin die neoplastischen Zellen, denen p53-Suppressorfunktion fehlt, Krebszellen von einem bronchogenen Karzinom, nasopharyngealen Karzinom, Kehlkopfkarzinom, Kleinzellen- und Nicht-Kleinzellen-Lungenkarzinom, Lungenadenokarzinom, Hepatokarzinom, Pankreaskarzinom, Blasenkarzinom, Darmkarzinom, Brustkarzinom, Zervixkarzinom, Eierstockkarzinom oder lymphozytischer Leukämie sind.

19. Verfahren nach einem der Ansprüche 16 bis 18, worin das Verfahren weiters das Kontaktieren der Zellpopulation mit einem Chemotherapeutikum umfaßt.

20. In vitro-Verfahren zum Detektieren der Abwesenheit funktioneller Tumorsuppressor-Aktivität in einer Zellpopulation, umfassend:
das Kontaktieren der Zellpopulation mit einem rekombinanten Adenovirus mit Replikationsdefizienz, dem ein exprimiertes virales Onkoprotein fehlt, das zur Bindung eines funktionellen p53-Tumorsuppressor-Genprodukts fähig ist, unter infektiösen Bedingungen, und das Bestimmen der Anzahl oder des Anteils an Zellen in der Population mit einem Replikationsphenotyp.

21. Verfahren nach Anspruch 20, worin das Bestimmen das Messen der Fähigkeit von Zellen, Farbstoffe auszuschließen, ein markiertes Nukleotid in virale DNA aufzunehmen oder Virione zu bilden, umfaßt.

## Revendications

1. Adénovirus déficient en réplication recombinante ne possédant pas une oncoprotéine virale exprimée capable de lier un produit de gène supresseur de tumeur p53 fonctionnel, pour utilisation dans une méthode de traitement ou de thérapie d'un mammifère;
ledit traitement ou ladite thérapie comprenant l'ablation des cellules néoplasiques dans une population de cellules comprenant lesdites cellules néoplasiques et des cellules non-néoplasiques;
lesdites cellules non-néoplasiques contenant un produit de gène supresseur de tumeur p53 fonctionnel capable de former un complexe lié avec ladite oncoprotéine virale et lesdites cellules néoplasiques ne possédant pas ledit produit de gène supresseur de tumeur p53 fonctionnel.

2. Adénovirus déficient en réplication recombinante pour utilisation selon la revendication 1 ne possédant pas une oncoprotéine E1b virale exprimée capable de lier un produit de gène supresseur de tumeur fonctionnel.

3. Adénovirus déficient en réplication recombinante pour utilisation selon la revendication 1 ou la revendication 2 où les cellules néoplasiques sont p53⁽⁻⁾RB⁽⁻⁾.

4. Adénovirus déficient en réplication recombinante pour utilisation selon l'une quelconque des revendications précédentes où l'adénovirus déficient en réplication recombinante n'encode pas un polypeptide E1a capable de lier RB et qui également n'encode pas un polypeptide E1b p55 capable de lier p53.

5. Adénovirus déficient en réplication recombinante pour utilisation selon la revendication 1, où ledit virus est un adénovirus du type 2 dl1520 ou un adénovirus de type 5 dl434.

6. Adénovirus déficient en réplication recombinante pour utilisation selon l'une quelconque des revendications précédentes où le mammifère est un humain.

7. Adénovirus déficient en réplication recombinante selon l'une quelconque des revendications précédentes où l'adénovirus est capable de former des virions infectieux dans la cellule néoplasique qui sont capables de se disséminer et d'infecter les cellules adjacentes in vivo chez un patient.

8. Adénovirus déficient en réplication recombinante pour utilisation selon l'une quelconque des revendications précédentes qui est non réplicable pour former des virions infectieux dans des cellules non-néoplasiques d'un patient humain.

9. Adénovirus déficient en réplication recombinante pour utilisation selon l'une quelconque des revendications précédentes comprenant de plus un gène de sélection négative ponté opérationnellement à un promoteur viral.

10. Adénovirus déficient en réplication recombinante pour utilisation selon l'une quelconque des revendications précédentes, où ledit traitement ou ladite thérapie comprend de plus la mise en contact desdites cellules avec un agent chimiothérapeutique.

11. Composition pharmaceutique comprenant un adénovirus déficient en réplication recombinante tel que défini dans l'une quelconque des revendications 1 à 10, et un porteur pharmaceutiquement acceptable.

12. Composition pharmaceutique selon la revendication 11 comprenant de plus un agent chimiothérapeutique.

13. Utilisation d'un adénovirus déficient en réplication recombinante ne possédant pas une oncoprotéine virale exprimée capable de lier un produit de gène supresseur de tumeur p53 fonctionnel, pour la fabrication d'un médicament pour l'ablation des cellules néoplasiques dans une population de cellules comprenant lesdites cellules néoplasiques et des cellules non néoplasiques;
lesdites cellules non néoplasiques contenant un produit de gène supresseur de tumeur p53 fonctionnel capable de former un complexe lié avec ladite oncoprotéine virale et lesdites cellules néoplasiques ne possédant pas ledit produit de gène supresseur de tumeur p53 fonctionnel.

14. Utilisation selon la revendication 13, où ledit adénovirus déficient en réplication recombinante est tel que défini dans l'une quelconque des revendications 2 à 10.

15. Utilisation selon la revendication 13 ou 14 pour le traitement d'un cancer négatif p53 sélectionné parmi un carcinome broncho-pulmonaire, un carcinome rhynopharyngien, un carcinome laryngien, un carcinome du poumon à petites cellules et à cellules non petites, un adénocarcinome du poumon, un hépatocarcinome, un carcinome pancréatique, un carcinome des reins, un carcinome du colon, un carcinome du sein, un carcinome cervical, un carcinome ovarien, ou une leucémie lymphocytique.

16. Méthode *in vitro* d'ablation de cellules néoplasiques ne possédant pas un produit de gène supresseur de tumeur p53 fonctionnel dans une population cellulaire de cellules néoplasiques et non-néoplasiques qui ont une fonction de supresseur de tumeur p53, ladite méthode comprenant :
la mise en contact sous des conditions infectieuses (1) d'un adénovirus déficient en réplication recombinante ne possédant pas une oncoprotéine virale exprimée capable de lier un produit de gène supresseur de tumeur p53 fonctionnel, avec (2) une population de cellules comprenant lesdites cellules néoplasiques et des cellules non-néoplasiques, pour générer ainsi une population de cellules infectées de sorte que l'ablation desdites cellules néo-plasiques se produisent.

17. Méthode selon la revendication 16 où ledit virus est tel que défini dans l'une quelconque des revendications 1 à 10.

18. Méthode selon la revendication 16 ou 17 où lesdites cellules néoplasiques n'ayant pas la fonction de supresseur de p53 sont des cellules cancéreuses provenant d'un carcinome broncho-pulmonaire, d'un carcinome rhynopharyngien, d'un carcinome laryngien, d'un carcinome du poumon à petites cellules et à cellules non petites, d'un adénocarcinome du poumon, d'un hépatocarcinome, d'un carcinome pancréatique, d'un carcinome des reins, d'un carcinome du colon, d'un carcinome du sein, d'un carcinome cervical, d'un carcinome des ovaires, ou d'une leucémie lymphocytique.

19. Méthode selon l'une quelconque des revendications 16 à 18 dans laquelle ladite méthode comprend de plus la mise en contact de ladite population de cellules avec un agent chimiothérapeutique.

20. Méthode *in vitro* de détection de l'absence d'une activité de supresseur de tumeur fonctionnel dans une population de cellules, comprenant :
la mise en contact de ladite population de cellules avec un adénovirus déficient en réplication recombinante ne possédant pas une oncoprotéine virale exprimée capable de lier un produit de gêne supresseur de tumeur p53 fonctionnel, sous des conditions infectieuses, et la détermination du nombre ou de la proportion de cellules dans ladite population avec un phénotype de réplication.

21. Méthode selon la revendication 20, dans laquelle ladite détermination comprend la mesure de la capacité des cellules à exclure un colorant, à incorporer un nucléotide marqué dans un ADN viral, ou à former des virions.
